# EUROPEAN PATENT APPLICATION

(11) **EP 4 670 656 A1**
(43) Date of publication of application: **31.12.2025**
(21) Application number: 24907115.0
(22) Date of filing: 29.11.2024
(51) Int. Cl.: A61B 17/70, A61B 17/90, A61F 2/44, A61F 2/46

(54) **SYSTEM FOR GUIDING INTERVERTEBRAL SPACER BETWEEN VERTEBRAL BODIES**

(30) Priority: 19.12.2023 JP 2023213705
(71) Applicant: Liga Ace Corporation, Shizuoka City, Shizuoka 420-0886 (JP)
(72) Inventor: SHINOZAKI Yoshio, SHIZUOKA-SHI Shizuoka 420-0886 (JP)
(74) Representative: VKK Patentanwälte PartG mbB
(86) International application number: PCT/JP2024/042345
(87) International publication number: WO 2025/134728

(57) **Abstract**

Provided is a system that is capable of easily placing an interbody spacer accurately in a predetermined position between vertebral bodies using the PLIF procedure, even in a narrow surgical field.

The system has an interbody spacer including a pair of contact surfaces to be in contact with vertebral bodies, a ventral surface which is a side surface on a ventral side having a convex shape in plan view and which connects the pair of contact surfaces on the ventral side, a ventral groove formed along the curved direction on the ventral surface, and an interbody spacer engagement portion having a radius of curvature smaller than a radius of curvature of the ventral surface, formed on a bottom surface of the ventral groove. The system has a guiding tool for guiding the interbody spacer to a predetermined position between the vertebral bodies, the guiding tool including a guide rail to be fitted into the interbody spacer engagement portion on a distal end side, a handle portion to be grasped by the surgeon outside the patient's body on a proximal end side, and a guide rail engagement portion on a distal side of the guide rail having a radius of curvature in plan view which is the same as the radius of curvature of the interbody spacer engagement portion in plan view.

## Description

### [Technical Field]

The present invention pertains to a system for guiding an interbody spacer between vertebral bodies.

### [Background Art]

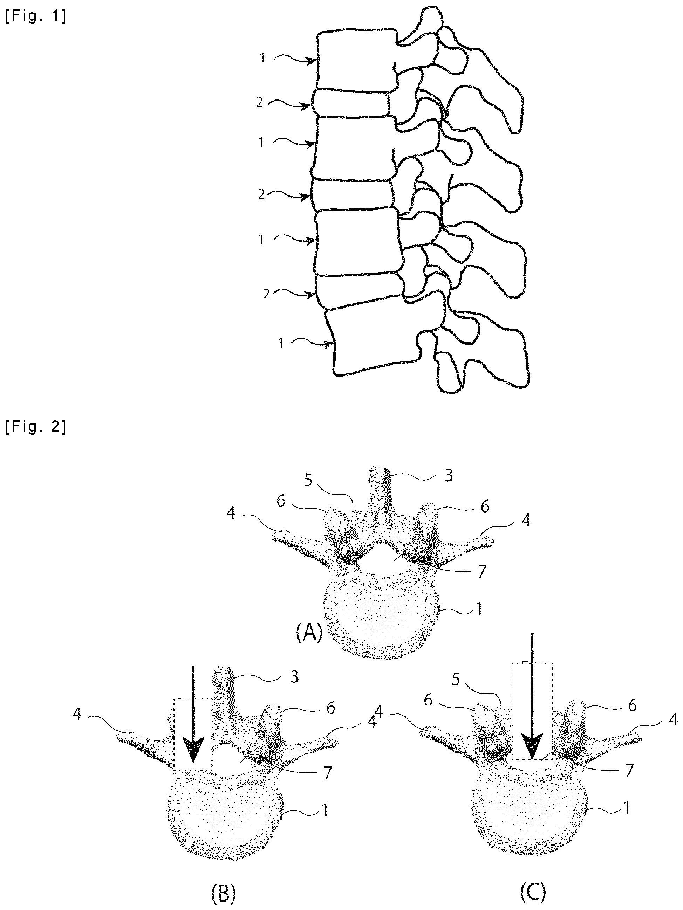

When inserting and placing an interbody spacer between vertebral bodies, fenestration is performed in the annulus fibrosus of an intervertebral disc 2 between vertebral bodies 1 shown in Fig. 1, and the nucleus pulposus therein is removed, and then the interbody spacer is inserted and placed in the intervertebral space after the removal. As the spinal nervous system and blood vessels run near the intervertebral disc 2, extreme care is required when carrying out the surgical operation.

As the spinal column performs the function of supporting body weight, an interbody spacer is required to have high rigidity and to cover a large area between vertebral bodies, and furthermore, as the surgical field is narrow and there are the nervous system and blood vessels running nearby, skills and surgical systems are required for placing the interbody spacer in an appropriate position while avoiding the nervous system and blood vessels.

Interbody spacers can be broadly classified into two types of shapes: a box-type interbody spacer and a boomerang-shaped interbody spacer. When comparing the bony fusion rate between interbody spacers and vertebral bodies, boomerang-shaped interbody spacers are superior to box-type interbody spacers.

Fig. 2(A) is a plan view of a lumbar spine, showing a spinous process 3, transverse processes 4, a vertebral arch 5, and intervertebral joints 6 above a vertebral body 1, with a spinal canal 7 between them. Spinal nerves pass through the spinal canal 7. The surgical procedures for placing an interbody spacer between vertebral bodies mainly include TLIF (Transforaminal Lumbar Interbody Fusion), shown in Fig. 2(B), and PLIF (Posterior Lumbar Interbody Fusion), shown in Fig. 2(C). TLIF is a surgical procedure in which either the left or right intervertebral joint 6 of the vertebral bone is resected to gain access, while PLIF is a surgical procedure in which the spinous process 3 in the central part of the vertebral bone is resected to gain access. In each of these procedures, the interbody spacer is inserted in the direction indicated by the arrow.

The system according to the invention described in Patent Literature 1 is a simple system capable of easily placing a boomerang-shaped interbody spacer accurately in a predetermined position between vertebral bodies, even in a narrow surgical field.

### [Prior Art Documents]

### [Patent Documents]

[Patent Literature 1] Japanese Patent No. 6700511

### [Summary of the Invention]

### [Problem to Be Solved by the Invention]

Among diseases of the spine such as the lumbar spine, the PLIF procedure is mainly used for lumbar spinal canal stenosis, and the TLIF procedure is mainly used for lumbar foraminal stenosis. Lumbar spinal canal stenosis is a common condition, and most experienced spinal surgeons are skilled at PLIF surgery. These surgeons tend to avoid TLIF surgery.

The system described in Patent Document 1 is based on the TLIF procedure. Therefore, placing a boomerang-shaped interbody spacer in a predetermined position between vertebral bodies using the PLIF procedure is difficult.

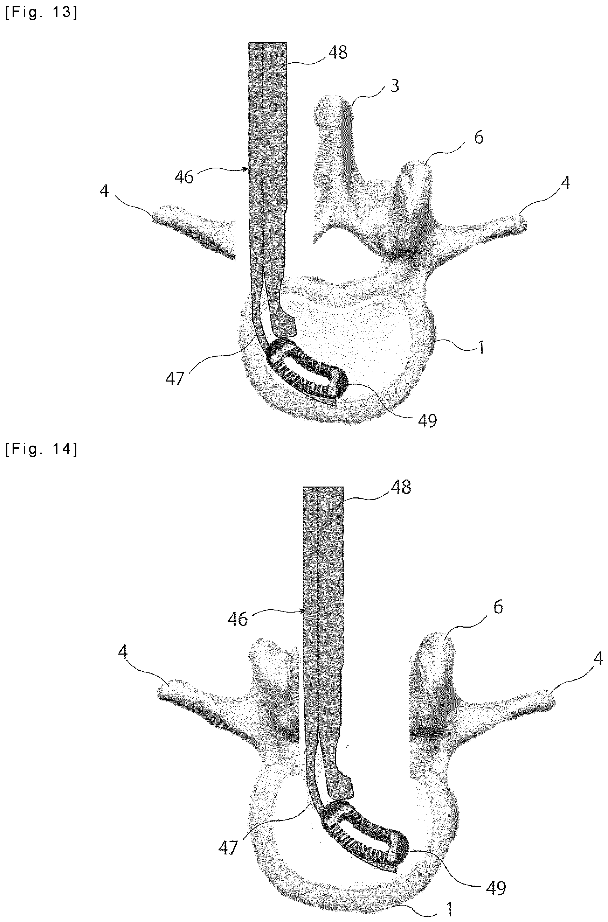

Fig. 13 shows the placement of a boomerang-shaped interbody spacer between vertebral bodies using the TLIF procedure. A conventional push rod 48 pushes an interbody spacer 49 that is engaged with a conventional guide rail engagement portion 47 of a conventional guide rail 46. In this case, the interbody spacer 49 can be easily placed in a predetermined position.

Fig. 14 shows the placement of a boomerang-shaped interbody spacer between vertebral bodies using the PLIF procedure in the invention described in Patent Document 1. Since the interbody spacer 49 moves along the conventional guide rail engagement portion 47, the tip of the interbody spacer 49 comes into contact with the side wall between the vertebral bodies, making it impossible to place the interbody spacer 49 in the predetermined position within the intervertebral space.

It is an object of the present invention to provide a simple system capable of easily placing an interbody spacer accurately in a predetermined position between vertebral bodies using the PLIF procedure, even in a narrow surgical field.

### [Means for Solving the Problem]

The present invention (1) is a system for treating a spinal disease, said system comprising: an interbody spacer which is curved so as to have a convex shape on a ventral side in plan view and used by being inserted between vertebral bodies, the interbody spacer including a pair of contact surfaces to be in contact with each of the vertebral bodies, a ventral surface which is a side surface on the ventral side which connects the pair of contact surfaces on the ventral side, a ventral groove formed on the ventral surface, and an interbody spacer engagement portion having a radius of curvature smaller than a radius of curvature of the ventral surface, formed on a bottom surface of the ventral groove; and a guiding tool for guiding the interbody spacer to a predetermined position between the vertebral bodies, the guiding tool including a guide rail to be fitted into the interbody spacer engagement portion on a distal end side, a handle portion to be grasped by the surgeon outside the patient's body on a proximal end side, and a guide rail engagement portion on a distal side of the guide rail having a radius of curvature in plan view which is the same as the radius of curvature of the interbody spacer engagement portion in plan view.

In the system of the present invention (1), the radius of curvature of the guide rail engagement portion on the distal side of the guide rail in plan view is the same as the radius of curvature of the interbody spacer engagement portion in plan view and smaller than the radius of curvature of the ventral surface in plan view. Therefore, the interbody spacer can be moved with good maneuverability, and a boomerang-shaped interbody spacer can easily be placed accurately in a predetermined position between vertebral bodies, even when using the PLIF procedure.

The present invention (2) is the system of the present invention (1), wherein a center of curvature of the interbody spacer engagement portion in plan view is eccentrically offset toward a tip side when inserting the interbody spacer between vertebral bodies, relative to a center of curvature of the ventral surface in plan view.

In the system of the present invention (2), the center of curvature of the interbody spacer engagement portion in plan view is eccentrically offset toward the tip side when inserting the interbody spacer between vertebral bodies, relative to the center of curvature of the ventral surface in plan view. Therefore, a boomerang-shaped interbody spacer can easily be placed in a predetermined position between vertebral bodies in surgical operation.

The present invention (3) is the system of the present invention (1) or (2), wherein a stopper portion is formed on the guide rail of the guiding tool to hook onto the intervertebral joint.

In the system of the present invention (3), the stopper portion is formed on the guide rail of the guiding tool to hook onto the intervertebral joint. Hooking the stopper onto the intervertebral joint prevents the guide rail from protruding beyond the predetermined position on the ventral side, enabling safer surgery.

The present invention (4) is the interbody spacer of the present inventions (2).

By using the interbody spacer of the present invention (4), a boomerang-shaped interbody spacer can easily be placed accurately in a predetermined position between vertebral bodies using the PLIF procedure, even in a narrow surgical field.

### [Effect of the Invention]

According to the system of the present invention, the system has a simple configuration, and thus it is possible to easily place a boomerang-shaped interbody spacer accurately in a predetermined position between vertebral bodies using the PLIF procedure, even in a narrow surgical field.

### [Brief Description of the Drawings]

[Fig. 1] shows a side view of the spine (the lumbar spine).
[Fig. 2] shows a plan view of the spine (the lumbar spine) (A), the approach direction of the TLIF procedure (B), and the approach direction of the PLIF procedure (C).
[Fig. 3] schematically shows the state in which an interbody spacer is attached to a guiding tool for an operation.
[Fig. 4] shows an enlarged view of the state in which the interbody spacer is attached to the guiding tool.
[Fig. 5] shows a perspective view of the interbody spacer according to the present invention.
[Fig. 6] shows a front view (A), a plan view (B), and a horizontal sectional view (C) of the interbody spacer according to the present invention.
[Fig. 7] schematically shows a comparison of the system of Example 1 according to the present invention (upper section) and the conventional interbody spacer described in Patent Document 1 (lower section).
[Fig. 8] schematically shows a comparison of the system of Example 1 (upper section) and the system of Example 2 (lower section) according to the present invention.
[Fig. 9] schematically shows the system according to the present invention in which the first interbody spacer is inserted.
[Fig. 10] schematically shows the system according to the present invention in which the second interbody spacer is inserted.
[Fig. 11] schematically shows the system of Example 3 according to the present invention in which a stopper is attached to the guiding tool.
[Fig. 12] schematically shows specific examples of the engagement state between the interbody spacer and a guide rail engagement portion of the guiding tool of the system according to the present invention.
[Fig. 13] schematically shows the system described in Patent Document 1 in which the interbody spacer is inserted between vertebral bodies using the TLIF procedure.
[Fig. 14] schematically shows the system described in Patent Document 1 in which the interbody spacer is inserted between vertebral bodies using the PLIF procedure.

### [Embodiment for Carrying Out the Invention]

The embodiment for carrying out the present invention will now be explained with reference to the drawings. In the following explanations, the superior and inferior denote an upper side and a lower side in the drawings, respectively. These "superior and inferior" are used for the sake of convenience, and upon placement, the superior and inferior may be reversed or the positioning may be horizontal.

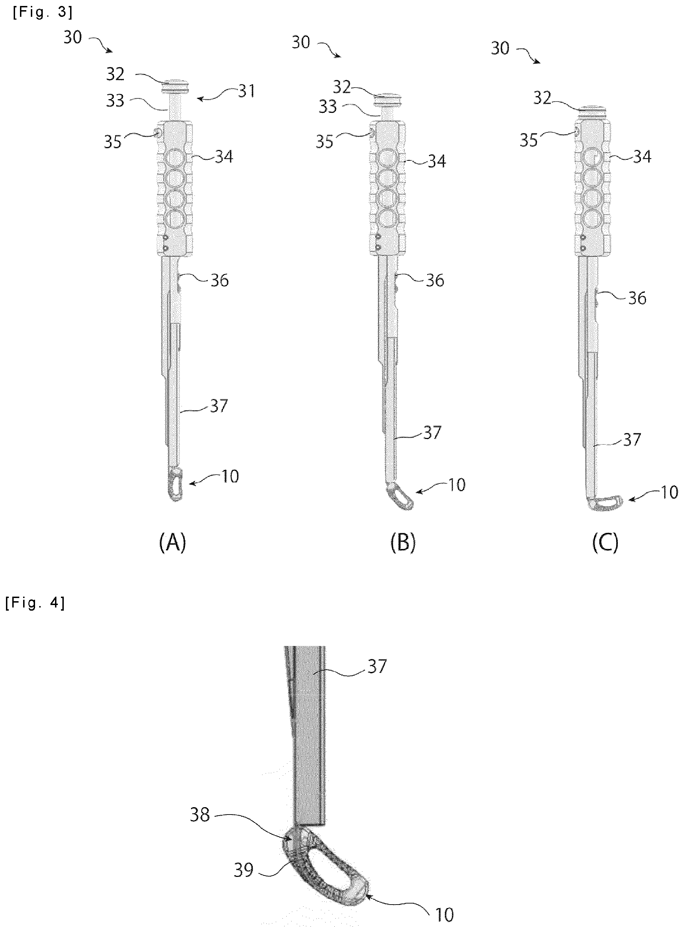

Fig. 3 shows a schematic view of the state in which an interbody spacer is attached to a guiding tool according to the present invention for an operation; an interbody spacer 10 is attached to a guiding tool 30 (A); the interbody spacer 10 is inserted approximately halfway into the predetermined position between vertebral bodies (B); and the interbody spacer 10 is removed from the guiding tool 30 after being placed in the predetermined position between vertebral bodies (C).

In Fig. 3(A), a head portion 32 of a push rod 31 is raised upward, and a rod portion 33 partly protrudes upward beyond an upper end of a handle portion 34. At this point, a push rod release button 35 protrudes and locks the push rod 31 in place so that it cannot move downward. The interbody spacer 10 is placed at the lower end of a push rod tip 37, and a gripping release button 36 is lowered so that the tip of a gripping rod (not numbered) embedded in the guiding tool 30 grips the interbody spacer 10.

Then, the interbody spacer 10 is inserted between vertebral bodies in the state shown in Fig. 3(A), in which the interbody spacer 10 is gripped. When the gripping release button 36 is pulled up, the gripping rod (not numbered) is released from the interbody spacer 10, thereby releasing the grip.

After that, the push rod release button 35 is pushed to press the head portion 32 downward, causing the push rod tip 37 to also move downward and press the upper end of the interbody spacer 10. The interbody spacer 10 is guided by the guide rail 38 (not shown in Fig. 3) and moves to the state shown in Fig. 3(B).

Fig. 3(C) shows a state in which the head portion 32 is pushed down to the upper surface of the handle portion 34 and the interbody spacer 10 is moved along the guide rail engagement portion 39. When the handle portion 34 is lifted upward in this state, the interbody spacer 10 disengages from the guide rail engagement portion 39, so only the handle portion 34 is removed from the body, and the interbody spacer 10 is placed in the predetermined position between vertebral bodies.

Fig. 4 shows an enlarged view of the state in which the interbody spacer 10 is attached to the guiding tool 30, and shows the stage of Fig. 3 (B).

It shows the push rod tip 37 and the guide rail 38, which has the guide rail engagement portion 39 at its tip. The interbody spacer 10 is gripped by the gripping rod (not numbered) provided inside the push rod tip 37.

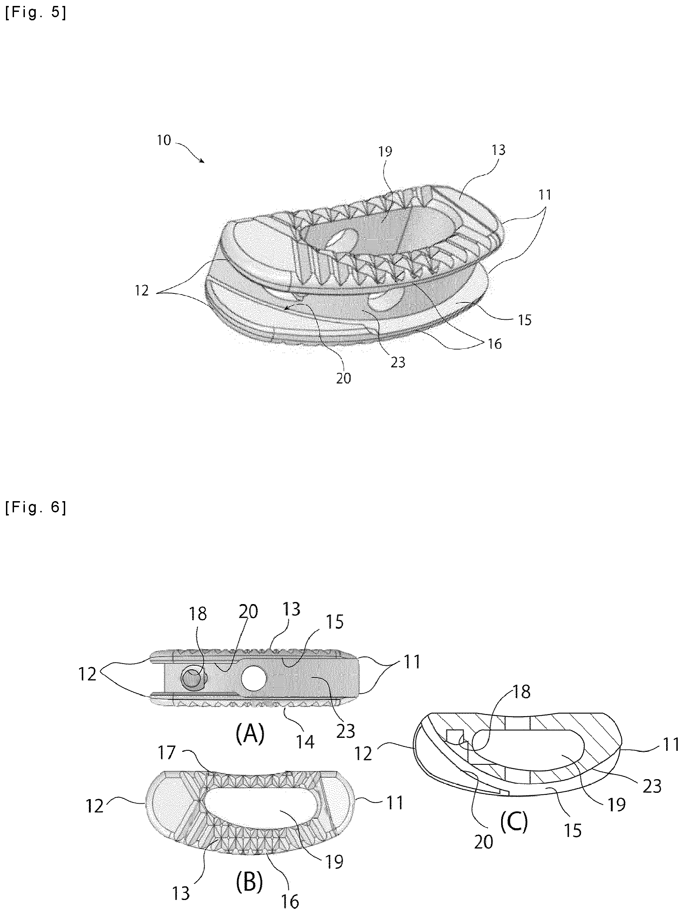

Fig. 5 shows a perspective view of the interbody spacer 10 according to the present invention. A ventral groove 15 is formed on a ventral surface 16, a bottom surface 23 is formed at the bottom of the ventral groove 15, and an interbody spacer engagement portion 20 is formed along the bottom surface 23. It engages with the guiding tool 30 on the side of a rear end face 12, and a tip face 11 is formed on the opposite side. The surface in contact with the cranial vertebral body in the intervertebral space is a superior surface 13, and the surface in contact with the caudal vertebral body in the intervertebral space is an inferior surface 14 (see Fig. 6). These contact surfaces have many deep grooves to promote bony fusion. The center of the interbody spacer 10 is open, forming a bone-graft site 19 into which bone grafts or hydroxyapatite can be inserted.

Fig. 6 shows a front view (A), a plan view (B), and a horizontal sectional view (C) of the interbody spacer according to the present invention. The explanation of the parts that overlap with Fig. 5 is omitted. (C) shows a gripping hole 18, which is a hole for engaging with the tip of the gripping rod (not numbered) of the guiding tool 30 in a horizontal sectional view.

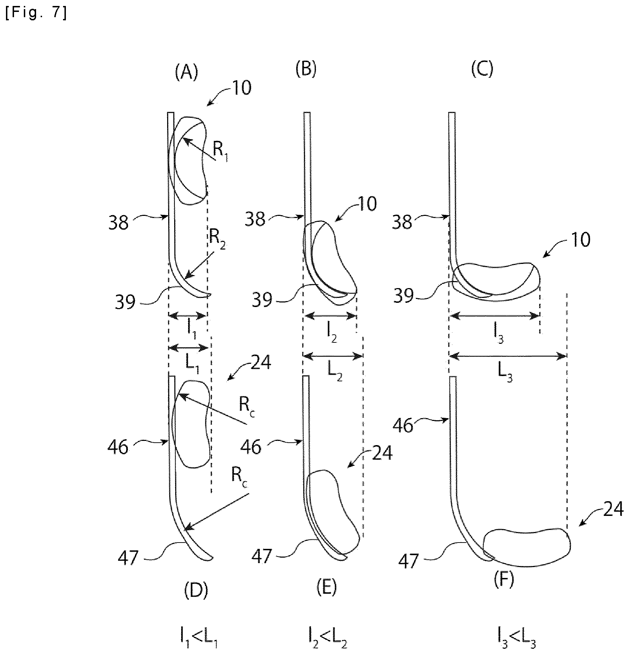

Fig. 7 schematically shows a comparison of the system of Example 1 (upper section) and the conventional interbody spacer system described in Patent Document 1 (lower section). (A) shows the interbody spacer 10 starting to move along the guide rail 38, (B) shows the interbody spacer 10 engaging with the guide rail engagement portion 39 and rotating, and (C) shows the finished placement of the interbody spacer 10 in the predetermined position between vertebral bodies. The radius of curvature of the interbody spacer engagement portion 20 is R₁.

(D) shows the conventional interbody spacer 24 starting to move along the conventional guide rail 46, (E) shows the conventional interbody spacer 24 engaging with the conventional guide rail engagement portion 47 and rotating, and (F) shows the finished placement of the conventional interbody spacer 24 in the predetermined position between vertebral bodies. The radius of curvature of the engagement portion is R_{C}.

The system of Example 1 of the present invention in the upper section is more maneuverable than the conventional interbody spacer system in the lower section. Therefore, when comparing the widths from the guide rail between I₁, I₂, I₃, and L₁, L₂, L₃, it can be seen that I₁ < L₁, I₂ < L₂, and I₃ < L₃. Consequently, the boomerang-shaped interbody spacer can easily be accurately inserted in a predetermined position between vertebral bodies with the system of Example 1 of the present invention, even when using the PLIF procedure.

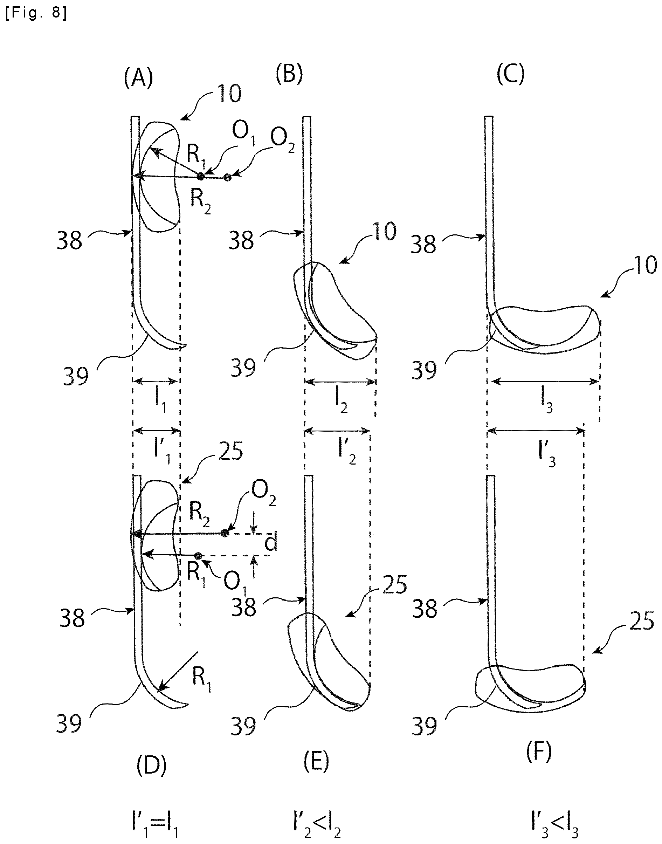

Fig. 8 schematically shows a comparison of the system of Example 1 (upper section) and the system of Example 2 (lower section) according to the present invention. The upper section (A), (B), and (C) are the same as the upper section (A), (B), and (C) in Fig. 7. The vertical position of the center of curvature O₁ of the engagement portion in the upper section is the same as the vertical position of the center of curvature O₂ of the ventral surface 15.

In the system of Example 2 in the lower section, the center of curvature O₁ of the interbody spacer engagement portion 20 and the center of curvature O₂ of the ventral surface 16 are not concentric, and the position of O₁ is shifted toward the tip by d. The widths from the guide rail are I₁' = I₁, I₂' < I₂, and I₃' < I₃. Consequently, it is easier to insert the boomerang-shaped interbody spacer in a predetermined position between vertebral bodies using the PLIF procedure with the system of Example 2 than with the system of Example 1.

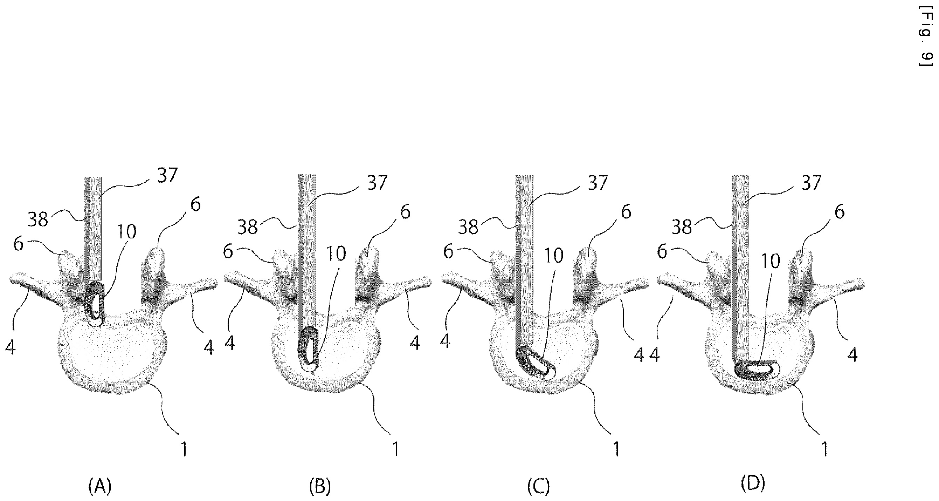

Fig. 9 schematically shows the system according to the present invention in which the first interbody spacer 10 is inserted. The interbody spacer 10 is inserted between vertebral bodies in the order of (A) to (B), (B) to (C), and (C) to (D), and are positioned in the predetermined position.

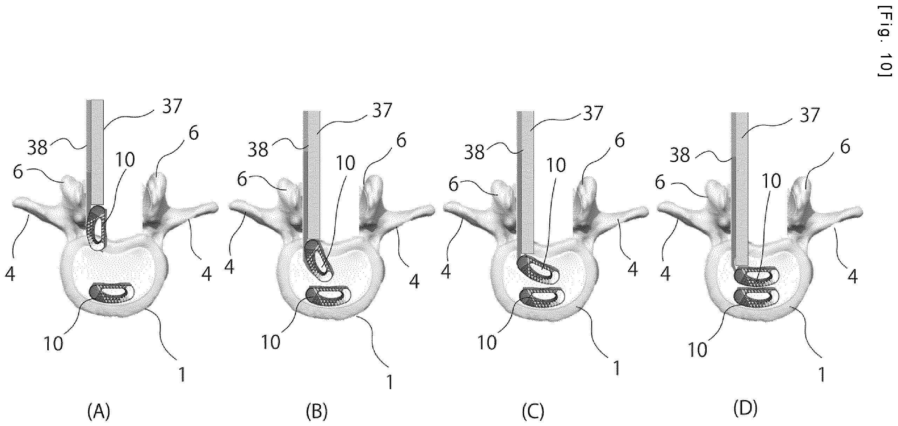

Fig. 10 shows the state in which the second interbody spacer 10 is sequentially placed following Fig. 9. As this figure shows, the first and second interbody spacers 10 can be placed easily and accurately in the predetermined position between vertebral bodies using the PLIF procedure.

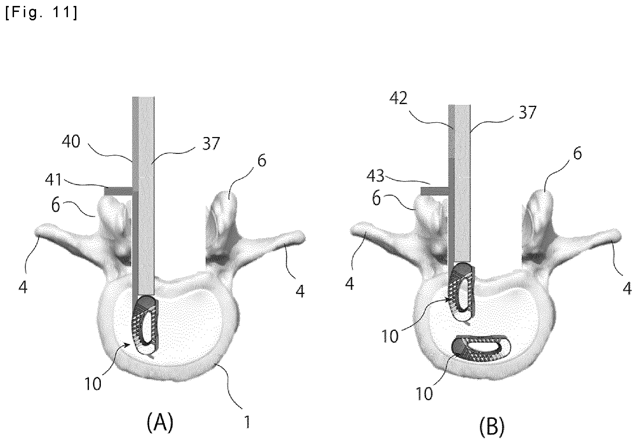

Fig. 11 schematically shows the system of Example 3 according to the present invention in which a stopper 41 is attached to the guiding tool 40. (A) shows the state in which the first interbody spacer 10 is inserted, and (B) shows the state in which the second interbody spacer 10 is inserted.

In (A), the interbody spacer 10 is engaged at the tip of the guide rail 40, and the push rod tip 37 presses against the rear end face 12 of the interbody spacer 10. The stopper 41 is formed at a predetermined position on the guide rail 40, and the stopper 41 is hooked onto the intervertebral joint 6. This stopper 41 prevents the guide rail 40 from protruding beyond the vertebral body 1.

(B) shows the state in which the second interbody spacer 10 is inserted. The guide rail 40 used in (A) is replaced with a guide rail 42. A stopper 43 is also formed on the guide rail 42, but it is closer to the tip than the stopper 41. This ensures that the first interbody spacer 10 is not pushed in incorrectly.

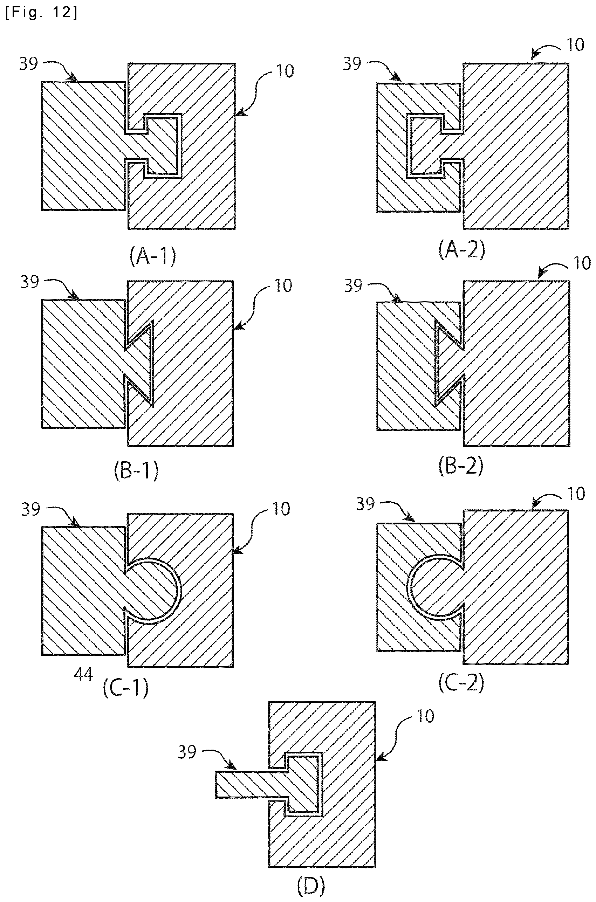

Fig. 12 schematically shows specific examples of the engagement state between the interbody spacer 10 and the guide rail engagement portion 39. In Fig. 12 (A-1), the cross-section of the guide rail engagement portion 39 is a T-shaped convex, and the cross-section of the opposing interbody spacer engagement portion is a T-shaped concave. (A-2) shows the reverse of Fig. 12 (A-1).

Fig. 12 (B-1) and Fig. 12 (B-2) show an engagement structure of a dovetail groove, and Fig. 12 (C-1) and Fig. 12 (C-2) show an engagement structure having a ball-and-socket relation. These engagements have their own characteristics, and the T-shape and the dovetail groove shape are characterized in having an advantage in the processing cost and capability of secure retention, while the ball-and-socket shape has a better sliding property.

Fig. 12 (D) is a modification of Fig. 12 (A-1), in which the height of the guide rail engagement portion 39 is extremely small compared to the height of the ventral surface of the interbody spacer, and the interbody spacer 10 can be more easily and accurately placed in a predetermined position even in a narrow area between vertebral bodies.

### [Industrial Applicability]

The system according to the present invention is a simple system, and an interbody spacer can easily be placed accurately in a predetermined position between vertebral bodies using the PLIF procedure, even in a narrow surgical field.

### [Descriptions of Reference Numerals]

1: vertebral body
2: intervertebral disc
3: spinous process
4: transverse process
5: vertebral arch
6: intervertebral joint
7: spinal canal
10: interbody spacer
11: tip face
12: rear end face
13: superior surface
14: inferior surface
15: ventral groove
16: ventral surface
17: dorsal surface
18: gripping hole
19: bone-graft site
20: interbody spacer engagement portion
23: bottom surface
24: conventional interbody spacer
30: guiding tool
31: push rod
32: head portion
33: rod portion
34: handle portion
35: push rod release button
36: gripping release button
37: push rod tip
38, 40, 42: guide rail
39: guide rail engagement portion
41, 43: stopper
46: conventional guide rail
47: conventional guide rail engagement portion
48: conventional push rod
O₁: center of curvature of interbody spacer engagement portion
O₂: center of curvature of ventral surface
R₁: radius of curvature of interbody spacer engagement portion
R₂: radius of curvature of ventral surface
R_{C}: radius of curvature of conventional guide rail engagement portion

## Claims

1. A system for treating a spinal disease, said system comprising:
an interbody spacer which is curved so as to have a convex shape on a ventral side in plan view and used by being inserted between vertebral bodies,
the interbody spacer including a pair of contact surfaces to be in contact with each of the vertebral bodies, a ventral surface which is a side surface on the ventral side which connects the pair of contact surfaces on the ventral side, a ventral groove formed on the ventral surface, and an interbody spacer engagement portion having a radius of curvature smaller than a radius of curvature of the ventral surface, formed on a bottom surface of the ventral groove; and
a guiding tool for guiding the interbody spacer to a predetermined position between the vertebral bodies,
the guiding tool including a guide rail to be fitted into the interbody spacer engagement portion on a distal end side, a handle portion to be grasped by the surgeon outside the patient's body on a proximal end side, and a guide rail engagement portion on a distal side of the guide rail having a radius of curvature in plan view which is the same as the radius of curvature of the interbody spacer engagement portion in plan view.

2. The system according to Claim 1, wherein the center of curvature of the interbody spacer engagement portion in plan view is eccentrically offset toward a tip side when inserting the interbody spacer between vertebral bodies, relative to the center of curvature of the ventral surface in plan view.

3. The system according to Claim 1 or 2, wherein a stopper portion is formed on the guide rail of the guiding tool to hook onto the intervertebral joint.

4. An interbody spacer according to Claim 2.
